# EUROPEAN PATENT SPECIFICATION

(11) **EP 1 407 738 B1**
(45) Date of publication and mention of the grant of the patent: **14.09.2011**
(21) Application number: 02771746.1
(22) Date of filing: 21.05.2002
(51) Int. Cl.: A61F 13/15, A61F 13/472, A61F 13/551

(54) **INTERLABIAL PAD AND PACKAGE THEREOF**
INTERLABIAL-POLSTER UND SEINE VERPACKUNG
ELEMENT INTERLABIAL ET ASSEMBLAGE DE CELUI-CI

(30) Priority: 22.05.2001 JP 2001152403; 17.10.2001 JP 2001319915
(43) Date of publication of application: 14.04.2004
(73) Proprietor: UNI-CHARM CORPORATION, Shikokuchuo-shi Ehime 799-0111 (JP)
(72) Inventor: MIZUTANI, Satoshi, Kagawa 769-1602 (JP); YAMAKI, Koichi, Kagawa 769-1602 (JP); NODA, Yuki, Kagawa 769-1602 (JP); TOKUMOTO, Megumi, Kagawa 769-1602 (JP); SAKAI, Akane, Kagawa 769-1602 (JP)
(74) Representative: Main, Malcolm Charles
(86) International application number: PCT/JP2002/004885
(87) International publication number: WO 2002/094149

(56) References cited:
- WO-A-99/55270
- WO-A1-98/08475
- WO-A1-98/57610
- WO-A1-99/01093
- WO-A1-99/01096
- WO-A1-99/26575
- US-A- 2 682 875
- US-A- 3 726 277
- US-A- 5 658 270
- US-A1- 2002 026 167

## Description

### Technical Field

The present invention relates to an interlabial pad used by fixation to the female labia, more specifically an interlabial pad which can be used with a sanitary napkin, and a wrapping body of said interlabial pad, wherein the interlabial pad is packaged in a wrapping container for individual wrapping.

### Background of Art

Conventionally, a sanitary napkin and a tampon are used generally as sanitary products for a female. However, there have been problems since as the sanitary napkin is used by bringing it into contact with clothes, it tends to cause the leak of menstrual blood from the gap caused by a poor contact state near the ostium vaginae, while the tampon tends to cause a foreign feeling and discomfort because of the nature of the product while wearing it, and it is difficult to fix it into the vagina.

In such a situation, a sanitary product of an interlabial pad has attracted people as a sanitary product positioned between the sanitary napkin and the tampon in recent years.

The interlabial pad is used by inserting its part between the labia and bringing it into contact with the labia, having advantages that it excels in a feeling of wearing and is comfortable because of being small as compared with the sanitary napkin, and it is sanitary and clean because the range of the body smeared with menstrual blood is narrow. Moreover, it has characteristics that it is difficult to cause the leakage of menstrual blood because of higher intimacy to the body than that of the sanitary napkin and psychological resistance to wearing is lower than that of the tampon which is inserted into the vagina.

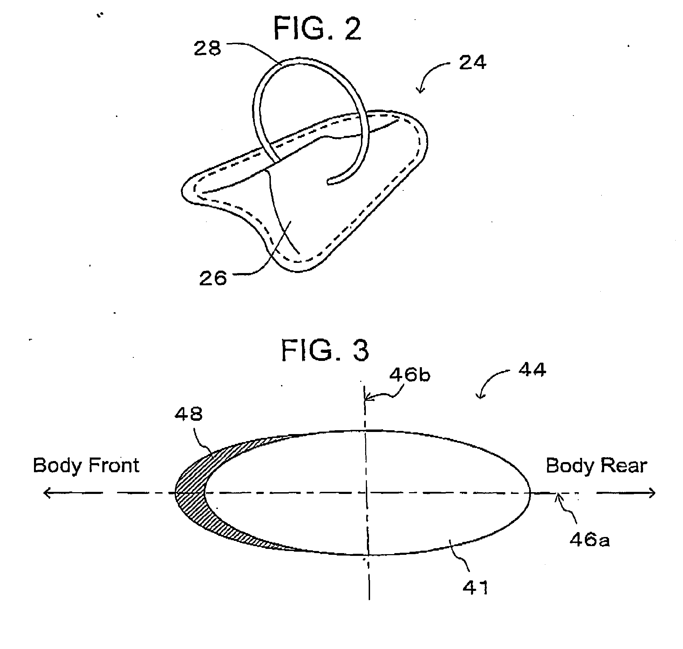

Incidentally, incontinence preventive devices which prevent incontinence in women are an example of a product used by fixation to the labia as in the case with the interlabial pad. Among incontinence preventive devices, to facilitate removal from between the labia after use by the wearer, for example, an incontinence preventive device 24 composed of an elastic main body 26 which closes the urethra as shown in Fig. 2, and has projections such as a ring 28 on the clothes-side (labial non-contacting side) is disclosed (Japanese Patent Publication No. 1994-506368).

The above mentioned incontinence preventive device has been designed to facilitate removal procedures by picking the projection on the clothes-side of the device when the wearer removes the device from between the labia after use. However, this device has problems as listed below.

Firstly, it is difficult for the wearer to visually identify the projection while wearing the device because the projection (ring 28) is attached near the center of the device on the clothes-side as shown in Fig. 2. In other words, the problem was that the wearer was forced to take an uncomfortable posture by bending forward in order to find the projection which is difficult to visually identify.

Secondly, the problem was that the wearer had to feel about the projection with the finger, due to the wearer's difficulty in visually identifying the projection. In other words, since the device cannot be removed without having a finger touch her labia where urine and body fluids are adherent, it is necessary and bothersome to wash her hands, and making her finger touch her labia also psychologically gives a feeling of lacking in sanitation.

Thirdly, the problem was that if rings and the like are to be attached to the clothes-side or opposite side of the device, it must be attached by penetrating through the device due to the shape of the attachment. In other words, although the opposite side of the device is generally comprised of a water impermeable material to prevent leakage of absorbed urine and body fluids to the clothes-side, the problem is that attachment of rings and the like results in opening a hole in said water impermeable material, thus leading to leakage. With this kind of device, it is not possible to simultaneously secure two basic functionalities, namely, cleanliness by not staining a wide portion of the wearer's skin and security of reducing attachment of stains to underwear.

The present invention was made after studying the above problems and its objective is not only simultaneously securing two basic functionalities, namely, cleanliness by not staining a wide portion of the wearer's skin and security of reducing attachment of stains to underwear, but also to provide an interlabial pad which can be quickly, easily, and also sanitarily removed from between the labia.

### Disclosure of the invention

To solve the above problem, in the state of fixation of the interlabial pad to the labia, the present invention provides a pull-up body for the interlabial pad, wherein said pull-up body projects outward from the interlabial pad, making it possible to not only simultaneously secure basic functionalities, namely, cleanliness by not staining a wide portion of the wearer's skin and security of reducing attachment of stains to underwear, but also to provide an interlabial pad which can be quickly, easily, and also sanitarily removed from between the labia.

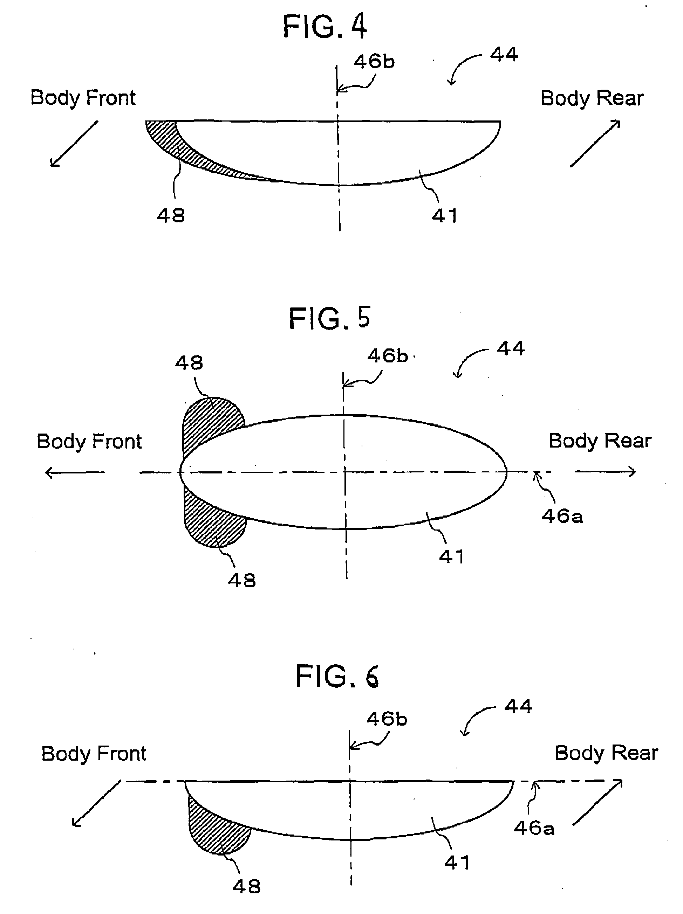

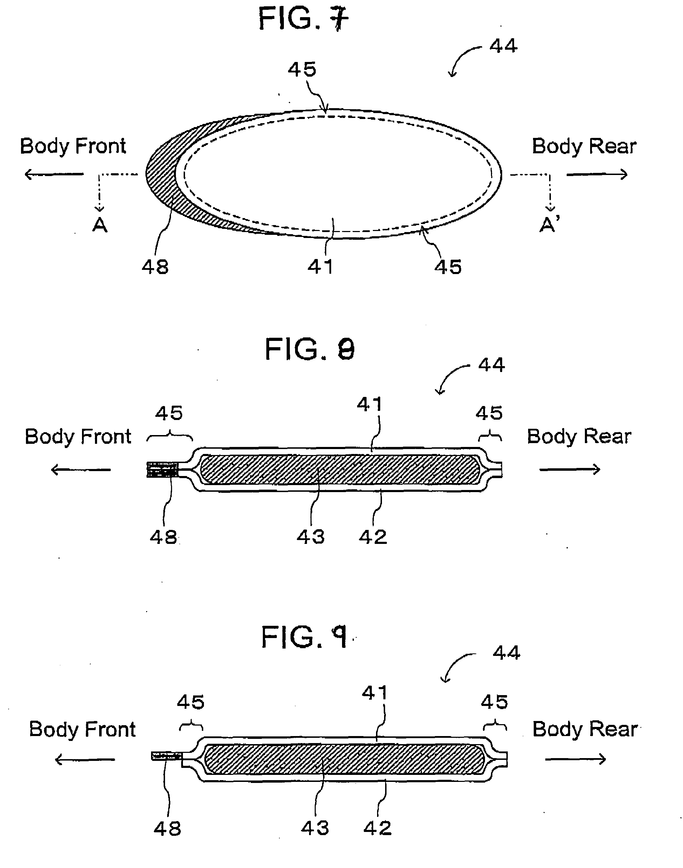

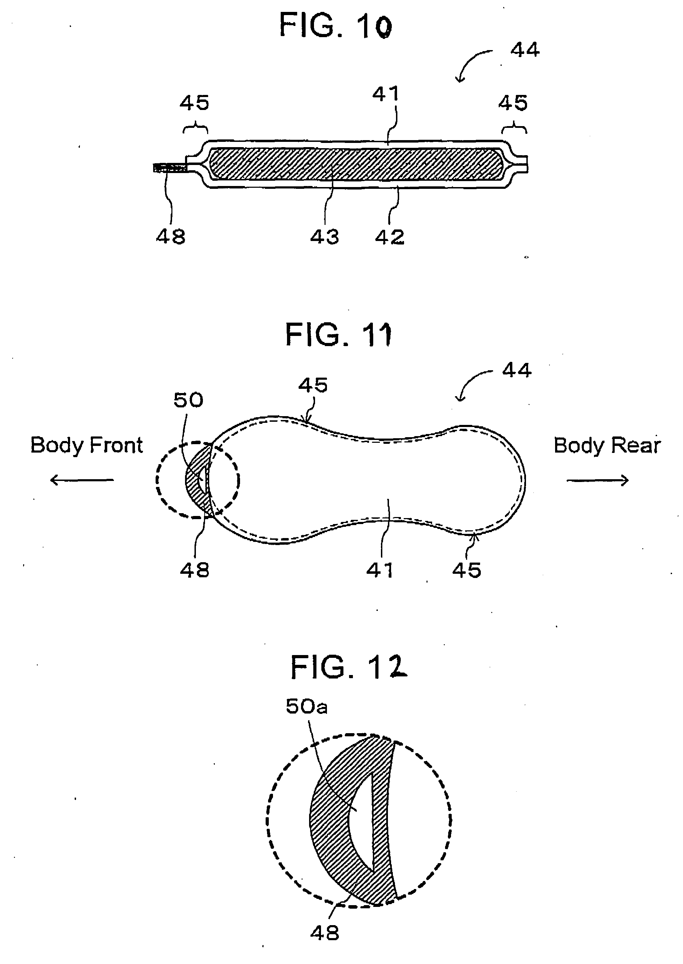

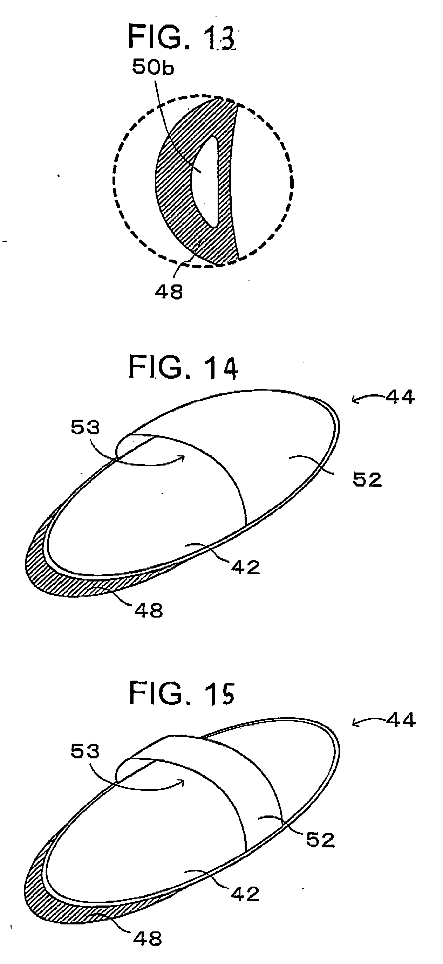

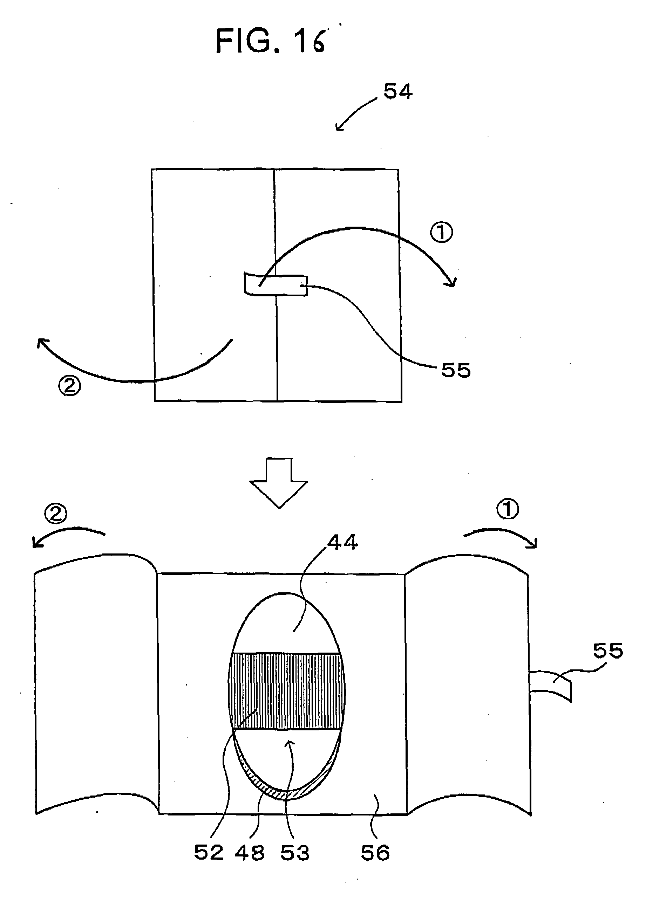

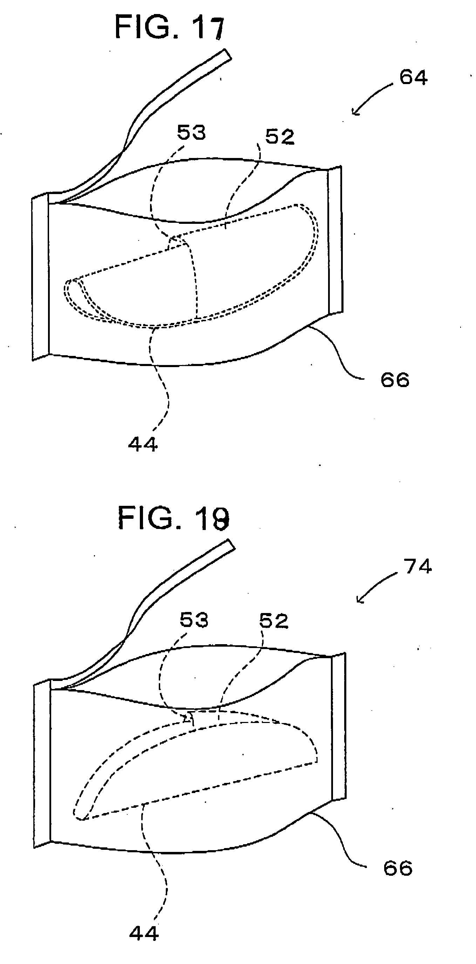

More specifically, the present invention provides the following.
(1) An interlabial pad as claimed in claim 1.
   The interlabial pad according to the present invention is provided with a pull-up body which projects from the interlabial pad, and when said interlabial pad is fixed to the labia, the pull-up body is exposed to the outside of the labia. Furthermore, the pull-up body is of sufficient size to pick with the fingers, which allows the wearer to easily remove the interlabial pad from between the labia by picking and pulling the pull-up body.
   Document WO 98/57610 discloses the features of the preamble of claim 1.
   Additionally, it is extremely easy for the wearer to visually identify the pull-up body and it is not necessary to feel about the pull-up body with the finger because the pull-up body is exposed to the outside of the labia. Thus, a situation where the labia covered with menstrual blood and body fluid contact a finger upon removal of the interlabial pad is prevented. Therefore, it is not needed to wash hands after removing the interlabial pad and the system does not give the wearer a psychological insanitation feeling.
   Furthermore, unlike a conventional ring or the like, the pull-up body can be attached without puncturing (without opening a hole in) the interlabial pad so that a situation leading to leakage can be avoided. Thus, it is possible to simultaneously retain basic functionalities, namely, cleanliness by not staining a wide portion of the wearer's skin and reduction of stains to underwear.
   According to the interlabial pad of the present invention, the pull-up body is provided to project from the front end of the interlabial pad (the region located near the wearer's clitoris upon wearing) Thus, during wearing the interlabial pad, said pull-up body is exposed as it projects from the wearer's labia toward the body front.
   As shown in Fig. 3 for example, the interlabial pad according to the present invention is composed of a water permeable covering sheet 41 and a water impermeable support sheet 42, wherein said covering sheet and support sheet are bonded together and enclose an absorbent body which absorbs menstrual blood. When the interlabial pad is worn, the pad is used by facing the covering sheet 41 to the body side, and facing the support sheet 42 to the clothing side. At the edge (the edge closer to the body front) of the longitudinal direction of the interlabial pad 44, the pull-up body 48 is shaped so that the pull-up body projects even toward the body front from the tip of the said edge.
   For the interlabial pad 44 as shown in Fig. 3 the pad is folded along a substantial center line 46a in the longitudinal direction (see Fig. 4) and fixed by being sandwiched between the labia. In this state where the pad is fixed to the labia, the pull-up body 48 is projecting from the labia toward the body front so that the pull-up body 48 may be easily identified visually by the wearer. Thus, when removing the interlabial pad 44, the wearer does not have to take an uncomfortable posture by bending forward in order to feel about the pull-up body 48 with a finger.
   The pull-up body according to the present invention is not limited to a pull-up body projecting from the tip of said edge toward the body front, said edge (the edge closer to the body front) being located on the interlabial pad 44 along the longitudinal direction, as shown Fig. 3 . For example, the pull-up body may include a projecting member from the side edge (of the interlabial pad) farther toward the side as shown in Fig. 5.
   When the interlabial pad 44 is fixed to the labia as shown in Fig. 6, the pad is folded along the longitudinal direction 46a, and sandwiched between the labia. Thus, the pull-up body 48 is exposed as it projects toward the body front, depending upon the wearing state of the interlabial pad 44 to the labia.
   The length (dimension of the projected direction) of the pull-up body 48 is is in the range of 10 to 60 mm, since the length of the pull-up body facilitates being picked by the wearer. The width (dimension in the direction perpendicular to the projection direction) of the pull-up body 48 is in the range from 10 to 20 mm.
(2) The interlabial pad according to (1) wherein the garment side surface of said cover sheet and the body side surface of said support sheet are bonded at a peripheral edge of said interlabial pad to form a peripheral edge bonding area; and wherein a part of said peripheral edge bonding area is to form said pull-up body.
   For example, as shown in Figs. 7 and 8, a portion of the peripheral bonding area 45 may be considered as a pull-up body 48 which projects toward the body front from the edge of the body front side of the interlabial pad 44, wherein said peripheral bonding area 45 is made by bonding the garment side surface of the covering sheet 41 and the body side surface of the support sheet 42 at the peripheral edge of the interlabial pad 44. This has the advantage that it is easier to form the pull-up body compared to separately attaching the pull-up body.
(3) The interlabial pad according to (1) or wherein at a peripheral edge of said interlabial pad, the garment side surface of said cover sheet and the body side surface of said support sheet are bonded to form a peripheral edge bonding area; and
   wherein said cover sheet is projected from said peripheral edge bonding area so that said projected cover sheet is to form said pull-up body.
   In a similar configuration of the pull-up body of the interlabial pad as described above in (2), the pull-up body may be formed by a projection of only the covering sheet, instead of a projection of the whole peripheral bonding area. For example, as shown in Fig. 9, the pull-up body 48 may be formed by a projection of the covering sheet 41 from the peripheral bonding area 45, wherein said projecting covering sheet 41 projects toward the body front from the edge of the body front side of the interlabial pad 44. This also has the advantage that it is easier to form the pull-up body compared to separately attaching the pull-up body.
(4) The interlabial pad according to (1) or wherein at a peripheral edge of said interlabial pad, the garment side surface of said cover sheet and the body side surface of said support sheet are bonded to form a peripheral edge bonding area; and
   wherein said support sheet is projected from said peripheral edge bonding area so that said projected support sheet is to form said pull-up body.
   In a similar configuration of the pull-up body of the interlabial pad as described above in (2), the pull-up body may be formed by a projection of only the support sheet, instead of a projection of the whole peripheral bonding area. For example, as shown in Fig. 10, the pull-up body 48 may be formed by a projection of the support sheet 42 from the peripheral bonding area 45, wherein said projecting support sheet 42 projects toward the body front from the edge of the body front side of the interlabial pad 44. The above-described embodiment can also obtain the same effect as the interlabial pad according to the above (2) or (3) can.
   The interlabial pad according to the present invention has a discontinuous part 50 formed as a part of the pull-up body 48, for example, as shown in Fig. 11. By forming a discontinuous part 50, oozing of menstrual blood absorbed by the interlabial pad 44 can be blocked by the discontinuous part 50 so that it is prevented from oozing out to the pull-up body 48, which may cause that a finger is stained by the blood when the interlabial pad 44 is removed.
   The discontinuous part comprises an opening portion or a cut portion formed in said pull-up body.
   A specific example of the discontinuous part is, for example, an opening portion 50a or a cut portion made in the pull-up body 48 as shown in Fig. 12. To prevent oozing of menstrual blood absorbed by the interlabial pad 44, the opening portion or cut portion is long in the lateral direction of the pad.
(5) The interlabial pad according to any one from (1) to (4), (7), wherein said opening portion or cut portion formed in said pull-up body is in a shape without a sharp corner.
   The opening portion or the incision portion made in the pull-up body may be in the form of having corners as in the opening portion 50a shown in Fig. 12, but a form of having no corners is preferable, for example as shown in the opening part 50b in Fig. 13. In other words, the pull-up body 48 is pulled toward the body front side upon removal of the interlabial pad 44 from between the labia. But in the case of the form having corners as the opening portion 50a is shown in Fig. 12, stress is concentrated at the corner part and the pull-up body may be torn from said corner part. Concerning this point, if the form (form having a curvature) has no corner in the opening part 50b as shown in Fig. 13, stress is deconcentrated so that such a situation can be prevented.
(6) The interlabial pad according to any one from (1) to (5), comprising a compressed area for controlling oozing of liquid formed near a boundary between said pull-up body and said peripheral edge bonding area.
   The term "compressed area" as used in this specification is defined as an area being localized near a boundary of the pull-up body and peripheral bonding area formed by compressing at least the covering sheet and support sheet. The compressed covering sheet and support sheet have high density, so as to achieve a controlling effect on liquid oozing. Therefore, it is possible to prevent menstrual blood from oozing to the pull-up body such that a situation that a finger is stained with the menstrual blood upon pulling the pull-up body to remove the pad is prevented.
   The compressed area can be formed by compressing at least the covering sheet and support sheet, for example, emboss processing (preferably heat emboss processing) and the like. The emboss processing patterns which form the compressed area may be for example, line-shaped, dot-shaped and the like. It is more preferable to be dot-shaped considering flexible fittability between the labia. The dot-shaped pattern may preferably have, for example, 0.3 to 1.0 mm² dot-shaped emboss processed portions arranged with 0.5 to 2 mm intervals.
   The compressed area may preferably have a length (a dimension of the pad in the longitudinal direction) in the range of 0.3 to 2.0 mm considering stiffness during wearing, a width (a contour dimension if the compressed area is formed along the peripheral edge of the absorbent body) in the range of 3 to 20 mm and considering flexible wearability between the labia.
(7) The interlabial pad according to any one from (1) to (6), comprising a mini sheet piece being bonded in at least one bonding area in each side in the longitudinal direction of said support sheet on the garment side of said support sheet and having at least one or more non-bonded area in the lateral direction of said support sheet; and a finger insertion opening which directly secures a fingerbreadth opening in the surface direction of said support sheet and is formed between the mini sheet piece and the support sheet by utilizing said at least one non-bonded area.
   According to the present invention, the interlabial pad may have a mini-sheet piece 52 attached to form a finger insertion opening 53 as shown in Figs. 14 and 15 for example. As shown in Figs. 14 and 15, in the mini-sheet piece 52, at least one sleeve portion of the two sleeve portions of the mini-sheet piece 52 along the lateral direction of said support sheet is a non-bonded portion to the surface of the support sheet 42. Thus, a sleeve opening is formed between the non-bonded sleeve portion of the mini-sheet 52 and the support sheet 42, so that the sleeve opening forms a finger insertion opening 53 where a finger may be inserted.
   Also, along the longitudinal direction of the support sheet 42, the mini-sheet piece 52 is only bonded on the right and left side part of the support sheet 42, and the inside is not bonded (attached) to the support sheet. Therefore, the mini-sheet piece 52 is attached in the state of bridging one side of the support sheet 42 to the other side so that a space which has both open ends or one closed end (finger insertion space) is formed at a hollow part where the mini sheet piece bridges from one side to the other side. Said space may be retained by inserting a finger.
   The term "finger breadth" as used in this specification is defined as not being the thickness of the finger, but specifically the width of the finger in the direction of the spread of the nail. The term "finger breadth opening" is defined as an opening which has a sufficient size to insert the finger.
   Additionally, to "directly secure" the finger breadth opening in the direction of the surface of the support sheet means that the pad itself is made to form primarily a shape suitable for finger insertion when the finger is inserted in a natural way to wear the pad (in a manner that the finger is inserted with the finger cushion facing the garment side surface of the support sheet). Thus, it may be excluded that a finger breadth opening is secondarily formed in the surface direction of the support sheet by turning the finger after the wearer inserts it.
   For the pad with a mini-sheet piece as described above, it is possible to temporarily fix and secure the pad at the fingertip by inserting the finger into said finger insertion opening. In this case, the finger insertion opening is formed as an opening of the finger width of the wearer and the flattened shape of the fingertip is naturally inserted tangentially to the surface of the support sheet without being inserted in a different direction from the support sheet. In other words, the finger insertion opening conforms to the shape of the wearer's fingertip and takes a widened shape in the direction of the support sheet surface so as to determine the finger insertion direction of the wearer and let the wearer search accurately for a wearing point with the finger cushion touching for the point. Thus, it makes it possible to apply the pad in the right position by locating the exact wearing point although it is difficult to wear the pad between the labia without the help of vision.
   Furthermore, in the present invention, the "side" of the longitudinal direction of the support sheet includes not only the peripheral edge portion of the pad but also the vicinity of the peripheral edge portion of the mini-sheet which may be bonded.
(8) The interlabial pad according to claim (7), wherein the pull-up body is a part of said mini sheet piece.
   As mentioned above, the pull-up body according to the present invention may be formed by projection of the entire peripheral edge bonding area or projection of only the covering sheet or the support sheet, but the pull-up body may also be formed by extension of part of the above mentioned mini-sheet piece. Compared to separately attaching the pull-up body, the advantage is that it is easier to form the pull-up body.
(9) An interlabial pad according to any one from (1) to (8), wherein the pad is an interlabial pad which is used together with a sanitary napkin.
   There have been users who use some sanitary napkins ( referred to as a "napkin" in the following description) laminated with each other when a large volume of menstrual blood is expected. However, there has been a problem of the bad wear feeling such as stiffness and the noticeability (remarkableness) of the pad from the outside of the garment. Furthermore, since the napkins are laminated at other than the vicinity of the ostium vaginae, which is the only place the laminated napkins are needed, rash or stuffy feeling may be caused. However, if the pad and the napkin are used together, the sanitary product is laminated only around the ostium vaginae. Thereby, the problem may be resolved. Further only the pad may be replaced without replacing the napkin so that the wearer does not have to carry a remarkable size of napkin. The term "sanitary napkin" as used in this specification includes not only a napkin sold for absorbing menstrual blood, but also a sheet for absorbing vaginal discharge.
(10) An interlabial pad according to any one from (1) to (9), wherein said interlabial pad is a pad for incontinence.
   According to the interlabial pad of the present invention, the pad may be used as an incontinence absorbance pad. Both the ostium vaginae where the menstrual blood is discharged and the urethral meatus where urine is discharged are located between the labia so that the interlabial pad between the labia can absorb urine according to the present invention.
   As described hereinbefore, the pad according to the present invention can absorb urine between the labia, especially around the urethral meatus so that it may be quite effective as an incontinence absorbing pad, especially, in the case of a light incontinence.
(11) An interlabial pad according to any one from (1) to (9), wherein said interlabial pad is a pad for absorbing vaginal discharge.
   In accordance with the invention, the interlabial pad can be used for a pad for absorbing vaginal discharge. Since the interlabial pad is used between labia and can absorb excretion (vaginal discharge) other than menstrual blood from the ostium vaginae, it may be used for such purpose (for absorbing vaginal discharge).
   As described above, the pad may absorb vaginal discharge in order to decrease the discomfort for the wearer, so that it is useful for the wearer who is not menstruating.
(12) An interlabial pad as recited in any one from (1) to (11), wherein said interlabial pad is enclosed in a wrapping container for individually wrapping the interlabial pad to form a wrapping body.
   Individual wrapping of the interlabial pad allows the wearer to carry a pad one by one (one by one individual wrapping body). In this embodiment, compared with the case of wrapping multiple pads in one wrapping container, each pad may be kept in a sanitary condition, and it is easier to carry one pad so as to ease handling.
(13) An interlabial pad as recited in any one of (7) to (12), wherein said interlabial pad is enclosed in a wrapping container for individual wrapping to form a wrapping body, the wrapping container being provided with an unwrapping portion, wherein
   said interlabial pad is enclosed in said wrapping container so that said finger insertion opening opens toward said unwrapping portion.
   The phrase "so that the finger insertion opening opens toward said unwrapping portion" means that, as shown in Fig. 16, it is enclosed so that, upon unwrapping the wrapping body 54, the mini-sheet piece 52, and the finger insertion opening which is formed by the mini-sheet piece is exposed such that the finger may be immediately inserted into the finger insertion opening 53. For example, the wrapping body 54 shown in Fig. 16 is unwrapped by pulling the tab tape 55 attached to the upper side of the wrapping container 56 toward the right of the figure, and the finger insertion opening 53 is exposed to the unwrapping portion so that the finger insertion opening opens toward the said unwrapping portion. Thus, the wearer may immediately insert the finger into the finger insertion opening 53.
(14) An interlabial pad according to (13), wherein said interlabial pad is enclosed in said wrapping container so that said mini sheet piece is folded toward the garment side along a substantially center line in the longitudinal direction of said interlabial pad.
   The phrase "folded toward the garment side" includes not only a state of the mini sheet piece folded completely with the garment side surface of the mini sheet piece outwards, but also a state of the mini sheet piece curved with the garment side surface of the mini sheet piece outwards. By thus enclosing the interlabial pad 44 in the wrapping container 66, the folded finger insertion opening 53 spontaneously opens upon unwrapping of the wrapping container 66, so that the wearer may easily identify the place to insert her finger so as to make application of the interlabial pad faster and easier.
   In this embodiment, provided that the mini-sheet piece is "folded toward the garment side", it is not required for the entire interlabial pad to be "folded toward the garment side". Therefore, for example, as shown in Fig. 17, in addition to the wrapping body 64 enclosing the entire interlabial pad 44, wherein said interlabial pad is "folded toward the garment side" to be enclosed in the wrapping container 66, wherein only the mini-sheet piece 52 may be "folded toward the garment side" as shown in Fig. 18, and according to the present invention, the wrapping body may include a wrapping body 74, wherein the main body of the interlabial pad 44 is folded backward from the garment side in the wrapping container 66 such that the garment side surface is folded inwardly.
   Additionally, the method of unwrapping the wrapping container is not particularly limited, as shown in Figs. 17 and 18. It may be unwrapped by cutting off the upper end of the wrapping container 66, or else, as shown in Fig. 19, it may be a wrapping container 86 that is unwrapped from the upper end to both sides (in a manner of a so-called "double doors").
(15) An interlabial pad according to any one of (1) to (14), wherein the interlabial pad is composed of biodegradable material and/or water dispersible and/or water soluble material.

### Brief Description of the Drawings

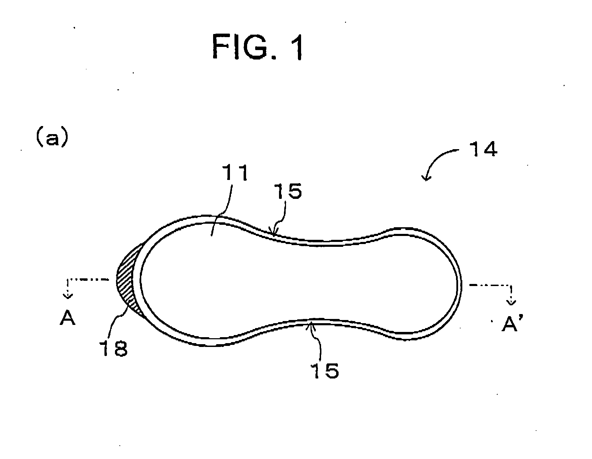

Fig. 1 shows the configuration of the interlabial pad according to the present invention. Fig. 1 (a) is a top view illustration. Fig. 2 is a perspective illustration showing the configuration of a conventional incontinence preventive device.
Fig. 3 is a top view illustration showing the configuration of the interlabial pad according to the present invention.
Fig. 4 is a side view illustration showing the configuration of the interlabial pad according to the present invention when it is folded.
Fig. 5 is a top view illustration showing the configuration of the interlabial pad according to the present invention.
Fig. 6 is a side view illustration showing the configuration of the interlabial pad according to the present invention when it is folded.
Fig. 7 is a top view illustration showing the configuration of the interlabial pad according to the present invention
Fig. 8 shows an A-A' cross-section of the interlabial pad shown in Fig. 7.
Fig. 9 shows a cross-section illustration showing the configuration of the interlabial pad according to the present invention.
Fig. 10 shows a cross-section illustration showing the configuration of the interlabial pad according to the present invention.
Fig. 11 is a top view illustration showing the configuration of the interlabial pad according to the present invention.
Fig. 12 is an enlarged view of the pull-up body of the interlabial pad according to the present invention.
Fig. 13 is an enlarged view of the pull-up body of the interlabial pad according to the present invention.
Fig. 14 is a perspective illustration showing the configuration of the interlabial pad according to the present invention.
Fig. 15 is a perspective illustration showing the configuration of the interlabial pad according to the present invention.
Fig. 16 is a process chart showing the procedures for opening the wrapping body according to the present invention.
Fig. 17 is a perspective illustration showing the configuration of the wrapping body according to the present invention.
Fig. 18 is a perspective illustration showing the configuration of the wrapping body according to the present invention.

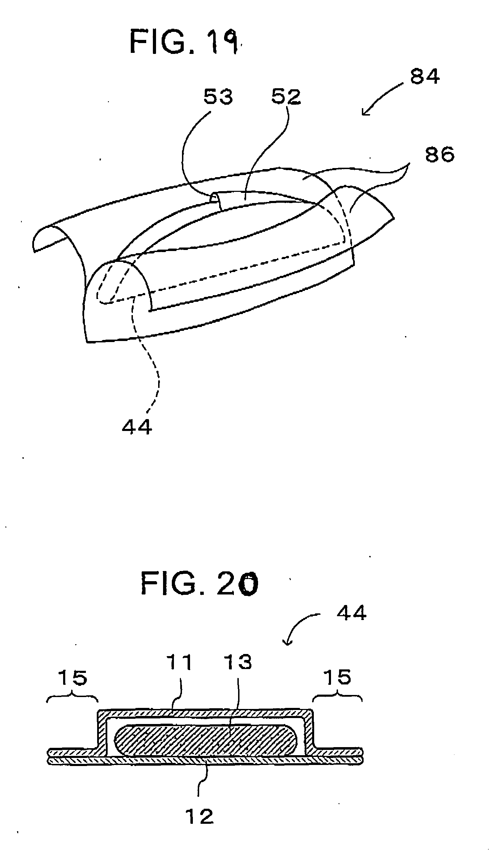

Fig. 19 is a perspective illustration showing the configuration of the wrapping body according to the present invention.
Fig. 20 is a cross-section showing the configuration of the interlabial pad according to the present invention.

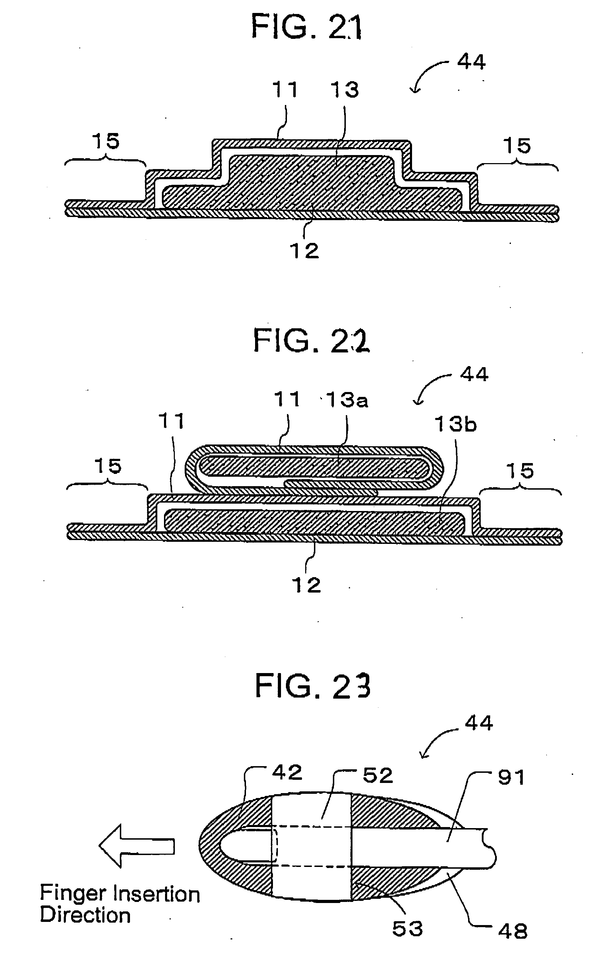

Fig. 21 is a cross-section showing the configuration of the interlabial pad according to the present invention.
Fig. 22 is a cross-section showing the configuration of the interlabial pad according to the present invention.
Fig. 23 is a top view illustration showing the embodiment of the mini-sheet piece of the interlabial pad according to the present invention.

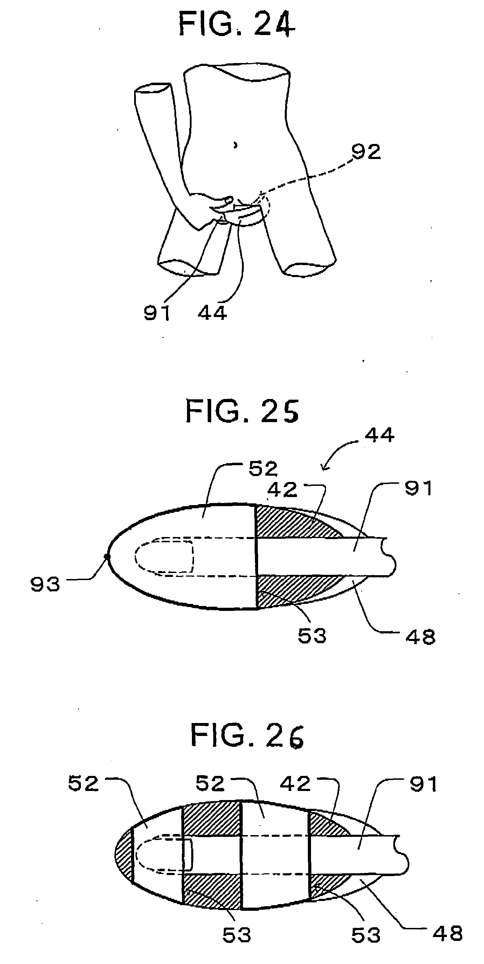

Fig. 24 is an explanatory illustration showing the procedures for application of the interlabial pad according to the present invention.
Fig. 25 is a top view illustration showing the embodiment of the mini-sheet piece of the interlabial pad according to the present invention.
Fig. 26 is a top view illustration showing the embodiment of the mini-sheet piece of the interlabial pad according to the present invention.

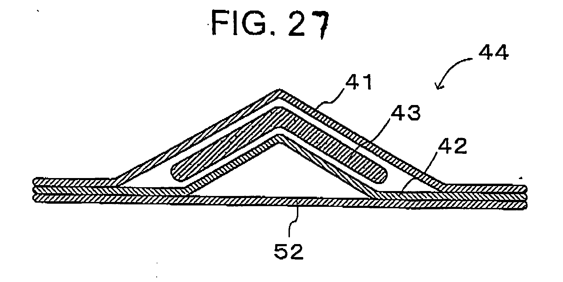

Fig. 27 is a cross-section showing the configuration of the interlabial pad according to the present invention.

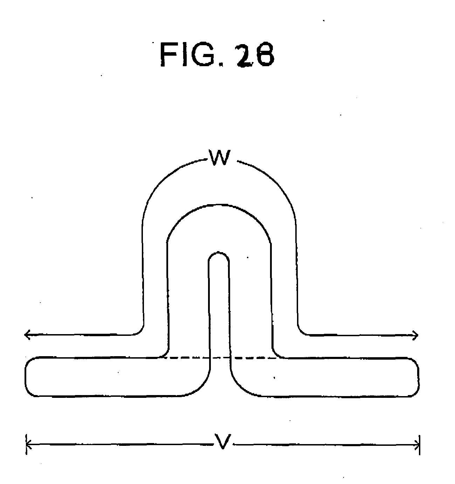

Fig. 28 is an illustration to explain the length dimension of the lateral direction of the interlabial pad.

### Best Mode of Carrying Out the Invention

Below are descriptions on examples of the preferred embodiments according to the present invention with referral to the illustrations. It is to be noted that in this specification, interlabial pad "width" refers to the dimension in the lateral direction of the interlabial pad, whereas interlabial pad "length" refers to the dimension in the longitudinal direction.

Fig. 1 shows the interlabial pad 14 according to the present invention. Fig. 1 (a) is a top view illustration.

### [(A) Basic composition of the interlabial pad]

As shown in Fig. 1 (a), the basic interlabial pad 14 according to the present invention is an embodiment formed by bonding a water permeable covering sheet 11 and water impermeable support sheet 12 and wherein the covering sheet and support sheet enclose an absorbent body 13 which absorbs menstrual blood. The covering sheet 11 and support sheet 12 are bonded at the peripheral edge portion of the pad, and this forms the peripheral edge bonding portion 15. Upon wearing, the covering sheet 11 is used while facing the body side, and the support sheet 12 is used while facing the garment side. Additionally, in the illustrated example, a pull-up body18 is adhered to the support sheet 12.

The pull-up body may be a pull-up body formed by extending the peripheral edge bonding portion of the covering sheet and support sheet, in which the pull-up body may be formed by extension of only the covering sheet and support sheet.

The shape of the pull-up body is not particularly limited, but it is necessary for the shape to prevent the finger from contacting a labia inner wall upon removal of the interlabial pad while picking the pull-up body. It is necessary that upon wearing the pad, the pull-up body projects forward out of the vaginal crevice so that the wearer may identify it directly by vision. Thus, as shown in the illustration, if the pull-up body18 is formed so that it projects even further toward the body front from the tip (an edge portion closer to the body front) of the peripheral edge in the longitudinal direction of the interlabial pad 14, the length is in the range of 10 to 60 mm. If the length is less than this range, the wearer cannot visually identify the pull-up body, which may lead to the fingers contacting the labial inner wall and the surrounding region of the labia. If the length exceeds this range, the pull-up body loses its shape during wearing, and there is a possibility for the pull-up body to become too hard to be pinched upon removal. Concerning easiness of pinching the body upon removal, the width is 10 to 20 mm.

The general shape of the interlabial pad 14 is not particularly limited as long as the pad 14 has a suitable shape for fitting itself between the labia. It is preferable that the shape is substantially elongated in the longitudinal direction. For example, an ellipsoid shape, an ovoid shape, a gourd shape, a tear drop shape, and the like may be applied.

The size of the interlabial pad 14 preferably should be determined after considering: easy insertion between the labia, retainability in the labia itself, and close contact without any gap between the labia and the interlabial pad. From such a viewpoint, the length is in the range of 80 to 150 mm, preferably in the range of 90 to 120 mm. If the length exceeds this range, a part of the interlabial pad may not be inserted between the labia, and the part may contact a sanitary napkin or underwear, such that it is likely that the interlabial pad falls out from between the labia. If the length is under this range, a gap between the interlabial pad and the labia may develop because the contact area between the labial inner wall and interlabial pad is reduced. Thus, it is more likely that the interlabial pad falls out from between the labia by an impact due to body pressure, a wearer's movements, and the like. Additionally, the apparent dimension in the lateral direction of the interlabial pad is in the range of 10 to 60 mm, preferably in the range of 20 to 40 mm. If the longitudinal dimension in the lateral direction is longer than 60 mm, a part which does not fit between the labia may rub against the wearer's femoral region, and there is possibility for the interlabial pad to fall out because a friction force may exceed the pinching force between the labia. Also, if the linear dimension in the lateral direction is less than 10 mm, the portion pinched between the labia is reduced so that the contact area with the inside of the labia is decreased and the possibility for the interlabial pad to fall out is increased. In addition, the above "apparent" means that the linear dimension is the minimal distance between two points

(corresponding to the V in Fig. 28). This is specifically defined because such distance may be measured by tracing between two points over the up-and-down surface, i.e., it may be the actual length, between the two points on the unfolded sheet (corresponding to the W in Fig. 28) in relation to the manufacturing process. Related to the thickness, considering comfortable wearability, thickness is preferably in the range of 2 to 10 mm, more preferably in the range of 3 to 6 mm.

The size of the absorption pad 13 enclosed by the covering sheet 11 and/or support sheet 12 is preferably made 2 to 10 mm smaller than the configuration outer line of the covering sheet11 and/or support sheet12, in order to prevent hardening of the fringe part and worsening of the wear feeling upon enclosure in the covering sheet11 and/or support sheet12.

An embodiment of the entire interlabial pad, for example as shown in Fig. 20, is a bonded type, wherein an absorbent body 13 is enclosed between a covering sheet 11 and a support sheet 12, and a peripheral edge bonding portion 15 is formed by bonding the covering sheet 11 and the support sheet 12 at the edge of the interlabial pad. In this embodiment, a water permeable material may lie between the covering sheet 11 and the absorbent body 13. Another embodiment, although not illustrated, is an encapsulated type and the like, wherein after arrangement of a support sheet under the absorbent body, it is formed by integrally wrapping a covering sheet around both components.

Yet another embodiment can have a three dimensional structure, wherein part of the absorbent body 13 is protruded toward the body-side for example, as shown in Fig. 21, or alternately have the three dimensional structure as shown in Fig. 22, wherein an upper absorbent body 13a and a lower absorbent body 13b, which have different widths, are stacked upon each other, and the front/back region or the contact region is bonded together, and other like embodiments. These embodiments are preferable because of their high conformity against the labial shape.

### [Covering sheet]

Since the covering sheet is the region which directly contacts the labial inner wall, it is preferably composed of material that does not aggravate the skin. For example, singular one or a mixture of nonwoven material obtained by production methods such as meltblown, spun bond, through-air, point bond, needle punch, spun lace and the like can be used. More specifically, a sheet made from one or an appropriate mixture of material selected from the group of rayon, acetate, natural cotton, pulp, or synthetic resin (singular fiber, or compound fiber with a sheath-core structure) is an example. Among these materials, considering liquid mobility from the inside of the labia and chemical stimulation by activators, and closeness with the labial inner wall, on the body-side, stacking 40 to 80% of rayon with fineness of 1.1 to 4.4dtex, fiber length of 7 to 51 mm, and on the clothes-side, spun lace nonwoven material with thickness adjusted in the range of 0.13 to 0.50 mm is preferable, wherein nonwoven material is a mixture composed of 14 to 42% of rayon with fineness of 1.1. to 4.4dtex and fiber length of 7 to 51 mm, against the total specific weight per unit area, and 6 to 18% of PET with fineness of 1.1. to 4.4dtex and fiber length of 7 to 51 mm, against the total specific weight per unit area are mixed, and after it is stacked so the total specific weight per unit area of the two layers is 20 to 60g/m², the fibers are tangled with each other using water flow passage and dried. In this case, by mixing PET on the clothes-side, it is possible to retain closeness with the labial inner wall because it is easier to keep bulkiness even if the water permeable sheet is in a wet state.

### [Absorbent body]

The absorbent body is preferably composed of a bulky material which does not easily lose its shape. For example, a mixture of material selected from the following group comprised of crushed pulp, rayon, acetate, natural cotton, air-laid pulp treated with chemical bonds, high water absorption polymer, superabsorbent fiber-like polymers, synthetic fibers, and the like. Additionally, the absorbent body may be made by filling the inside of the interlabial pad with the before mentioned covering sheet.

The absorbent body may be any material provided that it can absorb and retain liquid (body fluid), but preferably is bulky, does not lose its shape easily, has low chemical stimulation, and has a high flexibility for fitness with the labia. As a specific example, wherein pulp with fiber length selected from the range of 1 to 10 mm is stacked in a thickness of 50 to 150g/m² on the garment face side, and a nonwoven fabric sheet on the body face side, wherein the bulkiness of said nonwoven fabric sheet is adjusted to 2 to 10 mm, preferably 3 to 5 mm, and is made into sheet form by stacking 150 to 250g/m² and dot-shaped embossing of a mixture, wherein said mixture comprises of 60 to 90% of rayon selected from fineness in the range of 1.1 to 4.4dtex, fiber length in the range of 20 to 51 mm, and 40 to 10% of cotton. This allows liquid to flow more easily from the body-side to the garment side and increases the absorption and retention. Furthermore, it is possible to absorb more liquid even more efficiently by laying a mesh spun lace nonwoven fabric made from rayon with 1.1 to 4.4dtex fineness and 25 to 51 mm fiber length which is adjusted to specific weight per unit area 15 to 40g/m² on the body-side of the above mentioned pulp layer because liquid can be induced to almost all of the pulp layer by the mesh spun lace by diffusion of liquid that has flowed from the body-side.

### [Support sheet]

The ingredient material used for the support sheet is a water impermeable material which can prevent leakage of menstrual blood retained in the absorbent body from the interlabial pad. Additionally, it is possible to reduce discomfort during application, by reducing sweat during application by forming said support sheet with a moisture permeable material.

Water impermeable material used for the support sheet can be for example, water impermeable film in sheet-form made from synthetic resin which is produced as a thin film, air permeable film, complex of nonwoven fabric with laminated film on the back, and the like. A specific example of a composition using water impermeable material is a composition mainly comprising low density polyethylene (LDPE) resin, where film with density in the range of 0.900 to 0.925g/cm³, and specific weight per area in the range of 15 to 30g/m². Said example takes into consideration flexibility that does not impair wearability. More preferably, by disposing protruding projection portions by emboss process on the above film, a contact rate may be decreased so that the friction resistance may be reduced in order to reduce the danger for the interlabial pad to fall off between the labia because of high friction when the water permeable sheet contacts itself, concomitantly used pads, underwear, or the like during application between the labia.

### [Mini-sheet piece]

The ingredient material used for the mini-sheet piece should be selected after considering enough strength so that the material will not be damaged upon insertion of the finger, and can be selected from the group comprising sheet-like nonwoven fabric, elastic stretch nonwoven fabric, film, foam film, elastic film, foam sheets, tissue paper, and the like used singularly or as laminated products of the above mentioned materials.

Example of embodiments of the mini-sheet piece include an embodiment as shown in Fig. 23, wherein belt-shaped mini-sheet pieces 52 are laterally disposed along the lateral direction of the interlabial pad on the garment side surface of the support sheet 42 which comprises the interlabial pad 44. In this embodiment, the mini-sheet piece 52 is fixed to both ends of the interlabial pad 44, forming an opening facing the longitudinal direction of the interlabial pad 44, which in other words, forms the finger insertion opening 53.

In the above mentioned embodiment, by insertion of the finger 91 into the finger insertion opening, in which the ball of the finger 91 keeps contact with the support sheet 42, as shown in Fig. 24, the longitudinal direction of the interlabial pad 44 and the direction of the vaginal crevice 92 face the same direction. Therefore, it is possible to reliably apply the interlabial pad by pushing the interlabial pad 44 into the inside of the labia with the ball of the finger 91.

Additionally, for example, as shown in Fig. 25, the mini-sheet piece may be composed so that the support sheet 42 constituting the interlabial pad 44 may be completely covered with the mini sheet piece from the substantial center region of the longitudinal direction of the interlabial pad 44 to one edge 93 of the ends of said longitudinal direction. In this embodiment, the tip of the finger 91 is prevented from becoming exposed from the mini-sheet piece 52, retaining the state of no contact between menstrual blood and the finger 91, and is preferable from the point of allowing sanitary handling.

In addition, for example, as shown in Fig. 26, interlabial pad 44 having a plurality of belt-shaped mini-sheet pieces 52 arranged with an interval also can prevent the tip of the finger 91 from becoming exposed from the mini-sheet piece 52, and thus can obtain about the same effect of good hygiene as the interlabial pad 44 shown in Fig. 25,

Additionally, upon attachment of the mini-sheet piece to the interlabial pad, for example as in Fig. 27, it is preferable to attach a mini-sheet piece 52 which can retain a three dimensional shape, wherein the entire interlabial pad 44 is folded in a mountain fold toward the body side along the substantial center line of the longitudinal direction. This kind of structure is preferable from the point of it being easier for the interlabial pad to fit to the shape of the labia.

### [Fusing method]

Each of the above mentioned ingredient materials are joined by singular or concomitant use of heat emboss process, ultrasonic sealing, or hot-melt type adhesive, and the like.

### [Wrapping container]

The wrapping container which wraps the interlabial pad of the present invention can be comprised from those that are already known in the art. For example, nonwoven fabric formed from PE (polyethylene), PP (polypropylene), PET (polyethylene terephthalate), and the like; film with approximately 15 to 60µm thickness, paper, or laminated material made by laminating these material, and the like are enumerated.

### [(B) Composition of an interlabial pad with biodegradability, water dispersibility, water solubility]

Preferably, the interlabial pad of the present invention is composed of biodegradable material and/or water dispersible material and/or water soluble material. Such an interlabial pad can be flushed by just dropping into the toilet after use, allowing easy and sanitary disposal of the interlabial pad, and also can aim to reduce wastes within the toilet.

In this specification, "biodegradability" means degradation by biodegradable bacteria represented by actinomycetes, under conditions in the presence of other microorganisms, and following natural life process, under anaerobic conditions or aerobic conditions, where substances are degraded into gases such as carbon dioxide, methane, and the like; water; and biomass, wherein biodegradability (biodegradation speed, biodegradation degree, and the like) of said substances rivals that of material found in nature such as fallen leaves, or synthetic polymers which are generally identified as having biodegradability under the same environment. "Water dispersibility" means the same as water degradability, where there is no effect from the limited amount of water (menstrual blood) upon use, whereas in conditions of large amounts of water or under water flow, the fibers are easily dispersible into at least small pieces which cannot clog the toilet plumbing. "Water solubility" means the property of not being affected by limited amount of water (menstrual blood) upon use, but being soluble in large amounts of water or under a flow of water.

### [Covering sheet]

As material for the covering sheet to confer biodegradability, water dispersibility, and water solubility, for example, wet spun lace nonwoven fabric with fibril length in the range of 1 to 1.5 mm is enumerated. Additionally, one selected from the group of biodegradable resin such as polylactic acid, polybutylene succinate, and the like, acetate or rayon, synthetic fibers and the like, or cane which is a continuous fiber and adjusted in the range of 50 to 300g/m² and defiberized may be used.

### [Absorbent body]

The same material as those used for the previously mentioned covering sheet can be used as material for conferring biodegradability, water dispersibility, and water solubility to the absorbent body. Additionally, nonwoven fabric sheet with bulkiness of 2 to 5 mm, specific weight per unit area 50 to 250g/m² made from stacked fiber which is a mixture of 60 to 90% rayon or acetate selected from fineness in the range of 1.1 to 6.6dtex, and 10 to 40% fiber-shaped polymer absorbent and made into a sheet by twining around a needling. If said nonwoven fabric sheet is composed with carboxymethylcellulose fiber, it is more preferable from the standpoint of improving biodegradability, water dispersibility, and water solubility.

### [Support sheet]

Materials which can be used to confer biodegradability, water dispersibility, and water solubility, and also liquid impermeability to the support sheet include PVA (polyvinyl alcohol) film, sheet of polyvinyl alcohol film with water repellent treatment such as silicon resin on one or both sides, starch film, film whose raw materials are biodegradable resin such as poly lactic acid or polybutylene succinate, and material made by laminating the mentioned material with tissue and the like.

### [Mini-sheet piece]

Materials which can be used to confer biodegradability, water dispersibility, and water solubility to the mini-sheet piece include sheets of biodegradable or water soluble resin made from poly lactic acid, polybutylene, PVA resin and the like.

### [Pull-up body]

Materials which can be used to confer biodegradability, water dispersibility, and water solubility to the mini-sheet are the same materials as those listed for the covering sheet and support sheet. Additionally, the same material as for the previously described mini-sheet piece may also be used.

### [Joining method]

Additionally, joining methods which can be used to confer biodegradability, water dispersibility, and water solubility include adhesion by adhesive agents mainly comprising thermoplastic PVA resin, or adhesion by gel mainly comprising starch glue, acrylic acid, and including cross-linking agents, plasticizer.

### [Wrapping container]

Wrapping container which can be used to confer biodegradability, water dispersibility, and water solubility include those composed of fiber sheets made of water soluble fibers, film which uses biodegradable or water soluble resins, or laminated material made from the above fiber sheet and film, laminated material made from the above film and tissue and the like.

### Industrial Applicability

As described above, in the present invention, in the state of wearing the interlabial pad to the labia, wherein a pull-up body which projects toward the frontal part of the body is included, it is possible not only to secure basic functionalities, namely cleanliness by not staining a wide portion of the wearer's skin and security of reducing attachment of stains to underwear, but also sanitary removal.

## Claims

1. An interlabial pad (14, 44) for insertion between labia, the interlabial pad (14, 44) comprising
a water permeable cover sheet (11,41) facing a body side;
an impermeable support sheet (12, 42) facing a garment side;
an absorbent body (13, 43) disposed in between the cover sheet (11,41) and the support sheet (12, 42), the cover sheet (11, 41) and the support sheet (12, 42) being bonded together; and
a pull-up body (18, 48) projecting from the interlabial pad (14, 44) for removal of the interlabial pad (14, 44) from between the labia; **characterised in that** the interlabial pad (14, 44) has a length of 80 to 150 mm and a minimal width of 10 to 60 mm in a lateral direction and the pull-up body (18, 48) is in a sheet form and is formed by an extension of a part of the interlabial pad (14, 44) projecting from a front end of the interlabial pad (14, 44), the pull-up body (18, 48) being exposed to the outside of the labia in use and having a length in a projected direction of 10 to 60 mm, a width in a perpendicular direction to the projected direction of 10 to 20 mm and an opening portion (50a) or a cut portion formed therein extending in the lateral direction of the interlabial pad (14, 44).

2. The interlabial pad (14, 44) according to claim 1 wherein the garment side surface of said cover sheet (11, 41) and the body side surface of said support sheet (12, 42) are bonded at a peripheral edge of said interlabial pad (14, 44) to form a peripheral edge bonding area (15, 45), a part of said peripheral edge bonding area (15, 45) forming the pull-up body (18, 48).

3. The interlabial pad (14, 44) according to claim 1 wherein at a peripheral edge of said interlabial pad (14, 44), the garment side surface of said cover sheet (11, 41) and the body side surface of said support sheet (12, 42) are bonded to form a peripheral edge bonding area (15, 45), the cover sheet (11, 41) projecting from the peripheral edge bonding area (15, 45) to form said pull-up body (18, 48).

4. The interlabial pad (14, 44) according to claim 1 wherein at a peripheral edge of said interlabial pad (14, 44), the garment side surface of said cover sheet (11, 41), and the body side surface of said support sheet (12, 42) are bonded to form a peripheral edge bonding area (15, 45) of said interlabial pad (14, 44), said support sheet (12, 42) projecting from said peripheral edge bonding area (15, 45) to form said pull-up body (18, 48).

5. The interlabial pad (14, 44) according to any one of claims 1 to 4 wherein said opening portion (50b) or said cut portion formed in said pull-up body (18, 48) is of a shape which does not have a sharp corner.

6. The interlabial pad (14, 44) according to any one of claims 1 to 5 comprising a compressed area for controlling oozing of liquid formed near a boundary between said pull-up body (18, 48) and said peripheral edge bonding area (15, 45).

7. The interlabial pad (14, 44) according to any one of claims 1 to 6 comprising
a mini sheet piece (52) bonded in at least one bonding area in each side in the longitudinal direction of said support sheet (12, 42) on the garment side of said support sheet (12, 42) and having at least one non-bonded area in the lateral direction of said support sheet (12, 42); and
a finger insertion opening (53) which provides a fingerbreadth opening in the surface direction of said support sheet (12, 42) and is formed between the mini sheet piece (52) and the support sheet (12, 42) by utilizing said at least one non-bonded area.

8. The interlabial pad (14, 44) according to claim 7 wherein the pull-up body (18, 48) is a part of said mini sheet piece (52).

9. An interlabial pad (14, 44) according to any one of claims 1 to 8 wherein the pad is an interlabial pad (14, 44) which is used together with a sanitary napkin.

10. An interlabial pad (14, 44) according to any one of claims 1 to 9 wherein said interlabial pad (14, 44) is a pad for incontinence.

11. An interlabial pad (14, 44) according to any one of claims 1 to 9 wherein said interlabial pad (14, 44) is a pad for absorbing vaginal discharge.

12. An interlabial pad (14, 44) according to any one of claims 1 to 11 wherein said interlabial pad (14, 44) is enclosed in a wrapping container (56) for individually wrapping the interlabial pad (14, 44) to form a wrapping body (54).

13. An interlabial pad (14, 44) according to any one of claims 7 to 12 wherein said interlabial pad (14, 44) is enclosed in a wrapping container (56) for individually wrapping the interlabial pad (14, 44) to form a wrapping body (54), the wrapping container (56) being provided with an unwrapping portion, said interlabial pad (14, 44) being enclosed in said wrapping container (56) such that said finger insertion opening (53) opens toward said unwrapping portion.

14. An interlabial pad (14, 44) as claimed in claim 13 wherein when the interlabial pad (14, 44) is enclosed in said wrapping container (66), the mini sheet piece (52) is folded toward the garment side along a substantially centre line in the longitudinal direction of said interlabial pad (14, 44).

15. An interlabial pad (14, 44) as claimed in any one of claims 1 to 14 wherein the interlabial pad (14, 44) is composed of biodegradable material and/or water dispersible material and/or water soluble material.

## Patentansprüche

1. Ein interlabiales Kissen (14, 44) zur Einführung zwischen Labien, wobei das interlabiale Kissen (14, 44) Folgendes beinhaltet
eine einer Körperseite zugewandte wasserdurchlässige Abdeckschicht (11, 41); eine einer Kleidungsseite zugewandte undurchlässige Stützschicht (12, 42);
einen absorbierenden Körper (13, 43), der zwischen der Abdeckschicht (11, 41) und der Stützschicht (12, 42) angeordnet ist, wobei die Abdeckschicht (11, 41) und die Stützschicht (12, 42) aneinander gebunden sind; und
einen von dem interlabialen Kissen (14, 44) vorstehenden Körper (18, 48) zum Heraufziehen zur Entfernung des interlabialen Kissens (14, 44) von zwischen den Labien;
**dadurch gekennzeichnet, dass** das interlabiale Kissen (14, 44) eine Länge von 80 bis 150 mm und eine minimale Weite von 10 bis 60 mm in einer lateralen Richtung aufweist und der Körper (18, 48) zum Heraufziehen in einer Schichtform vorliegt und durch eine Erweiterung eines Teils des interlabialen Kissens (14, 44), vorstehend von einem vorderen Ende des interlabialen Kissens (14, 44), gebildet ist, wobei der Körper (18, 48) zum Heraufziehen bei Verwendung auf der Außenseite der Labien positioniert ist und in einer vorstehenden Richtung eine Länge von 10 bis 60 mm, in einer senkrechten Richtung zu der vorstehenden Richtung eine Weite von 10 bis 20 mm und einen darin gebildeten Öffnungsabschnitt (50a) oder einen Schnittabschnitt, der sich in die laterale Richtung des interlabialen Kissens (14, 44) erstreckt, aufweist.

2. Interlabiales Kissen (14, 44) gemäß Anspruch 1, wobei die Kleidungsseitenoberfläche der Abdeckschicht (11, 41) und die Körperseitenoberfläche der Stützschicht (12, 42) an einer Umfangskante des interlabialen Kissens (14, 44) gebunden sind, um einen Umfangskantenbindungsbereich (15, 45) zu bilden, wobei ein Teil des Umfangskantenbindungsbereichs (15, 45) den Körper (18, 48) zum Heraufziehen bildet.

3. Interlabiales Kissen (14, 44) gemäß Anspruch 1, wobei die Kleidungsseitenoberfläche der Abdeckschicht (11, 41) und die Körperseitenoberfläche der Stützschicht (12, 42) an einer Umfangskante des interlabialen Kissens (14, 44) gebunden sind, um einen Umfangskantenbindungsbereich (15, 45) zu bilden, wobei die Abdeckschicht (11, 41) von dem Umfangskantenbindungsbereich (15, 45) vorsteht, um den Körper (18, 48) zum Heraufziehen zu bilden.

4. Interlabiales Kissen (14, 44) gemäß Anspruch 1, wobei die Kleidungsseitenoberfläche der Abdeckschicht (11, 41) und die Körperseitenoberfläche der Stützschicht (12, 42) an einer Umfangskante des interlabialen Kissens (14, 44) gebunden sind, um einen Umfangskantenbindungsbereich (15, 45) des interlabialen Kissens (14, 44) zu bilden, wobei die Stützschicht (12, 42) von dem Umfangskantenbindungsbereich (15, 45) vorsteht, um den Körper (18, 48) zum Heraufziehen zu bilden.

5. Interlabiales Kissen (14, 44) gemäß einem der Ansprüche 1 bis 4, wobei der Öffnungsabschnitt (50b) oder der Schnittabschnitt, die in dem Körper (18, 48) zum Heraufziehen gebildet sind, von einer Form ist, die keine spitze Ecke aufweist.

6. Interlabiales Kissen (14, 44) gemäß einem der Ansprüche 1 bis 5, das einen komprimierten Bereich zum Kontrollieren des Durchsickerns von Flüssigkeit, der nahe einer Grenze zwischen dem Körper (18, 48) zum Heraufziehen und dem Umfangskantenbindungsbereich (15, 45) gebildet ist, beinhaltet.

7. Interlabiales Kissen (14, 44) gemäß einem der Ansprüche 1 bis 6, das Folgendes beinhaltet
ein Minischichtstück (52), das in mindestens einem Bindungsbereich in jeder Seite in der Längsrichtung der Stützschicht (12, 42) auf der Kleidungsseite der Stützschicht (12, 42) gebunden ist und in der lateralen Richtung der Stützschicht (12, 42) mindestens einen nicht gebundenen Bereich aufweist; und
eine Fingereinführungsöffnung (53), die in der Oberflächenrichtung der Stützschicht (12, 42) eine fingerbreite Öffnung bereitstellt und durch Benutzung des mindestens einen nicht gebundenen Bereichs zwischen dem Minischichtstück (52) und der Stützschicht (12, 42) gebildet ist.

8. Interlabiales Kissen (14, 44) gemäß Anspruch 7, wobei der Körper (18, 48) zum Heraufziehen ein Teil des Minischichtstücks (52) ist.

9. Interlabiales Kissen (14, 44) gemäß einem der Ansprüche 1 bis 8, wobei das Kissen ein interlabiales Kissen (14, 44) ist, das zusammen mit einer Damenbinde verwendet wird.

10. Interlabiales Kissen (14, 44) gemäß einem der Ansprüche 1 bis 9, wobei das interlabiale Kissen (14, 44) ein Kissen für Inkontinenz ist.

11. Interlabiales Kissen (14, 44) gemäß einem der Ansprüche 1 bis 9, wobei das interlabiale Kissen (14, 44) ein Kissen zum Absorbieren von Scheidenausfluss ist.

12. Interlabiales Kissen (14, 44) gemäß einem der Ansprüche 1 bis 11, wobei das interlabiale Kissen (14, 44) in einem Verpackungsbehälter (56) zum einzelnen Verpacken des interlabialen Kissens (14, 44) zum Bilden eines Verpackungskörpers (54) eingeschlossen ist.

13. Interlabiales Kissen (14, 44) gemäß einem der Ansprüche 7 bis 12, wobei das interlabiale Kissen (14, 44) in einem Verpackungsbehälter (56) zum einzelnen Verpacken des interlabialen Kissens (14, 44) zum Bilden eines Verpackungskörpers (54) eingeschlossen ist, wobei der Verpackungsbehälter (56) mit einem Auspackabschnitt bereitgestellt ist, wobei das interlabiale Kissen (14, 44) so in dem Verpackungsbehälter (56) eingeschlossen ist, dass sich die Fingereinführungsöffnung (53) zu dem Auspackabschnitt hin öffnet.

14. Interlabiales Kissen (14, 44) gemäß Anspruch 13, wobei, wenn das interlabiale Kissen (14, 44) in dem Verpackungsbehälter (66) eingeschlossen ist, das Minischichtstück (52) entlang einer im Wesentlichen Mittellinie in der Längsrichtung des interlabialen Kissens (14, 44) zu der Kleidungsseite hin gefaltet ist.

15. Interlabiales Kissen (14, 44) gemäß einem der Ansprüche 1 bis 14, wobei das interlabiale Kissen (14, 44) aus biologisch abbaubarem Material und/oder wasserdispergierbarem Material und/oder wasserlöslichem Material besteht.

## Revendications

1. Une protection interlabiale (14, 44) destinée à être insérée entre les lèvres génitales, la protection interlabiale (14, 44) comprenant
un feuillet de couverture perméable à l'eau (11, 41) tourné côté corps ;
un feuillet formant couche dorsale imperméable (12, 42) tourné côté vêtement ;
un corps absorbant (13, 43) disposé entre le feuillet de couverture (11, 41) et le feuillet formant couche dorsale (12, 42), le feuillet de couverture (11, 41) et le feuillet formant couche dorsale (12, 42) étant collés ensemble ; et
un corps formant tirette (18, 48) se projetant depuis la protection interlabiale (14, 44) destiné au retrait de la protection interlabiale (14, 44) d'entre les lèvres génitales ;
**caractérisée en ce que** la protection interlabiale (14, 44) a une longueur allant de 80 à 150 mm et une largeur minimale allant de 10 à 60 mm dans une direction latérale et le corps formant tirette (18, 48) est en forme de feuillet et est formé par une extension d'une partie de la protection interlabiale (14, 44) se projetant depuis une extrémité avant de la protection interlabiale (14, 44), le corps formant tirette (18, 48) étant exposé à l'extérieur des lèvres génitales lors de l'utilisation et ayant une longueur dans une direction de projection allant de 10 à 60 mm, une largeur dans une direction perpendiculaire à la direction de projection allant de 10 à 20 mm et une portion d'ouverture (50a) ou une portion découpée formée dans celui-ci s'étendant dans la direction latérale de la protection interlabiale (14, 44).

2. La protection interlabiale (14, 44) selon la revendication 1 dans laquelle la surface côté vêtement dudit feuillet de couverture (11, 41) et la surface côté corps dudit feuillet formant couche dorsale (12, 42) sont collées au niveau d'un bord périphérique de ladite protection interlabiale (14, 44) pour former une zone de collage de bord périphérique (15, 45), une partie de ladite zone de collage de bord périphérique (15, 45) formant le corps formant tirette (18, 48).

3. La protection interlabiale (14, 44) selon la revendication 1 dans laquelle au niveau d'un bord périphérique de ladite protection interlabiale (14, 44), la surface côté vêtement dudit feuillet de couverture (11, 41) et la surface côté corps dudit feuillet formant couche dorsale (12, 42) sont collées pour former une zone de collage de bord périphérique (15, 45), le feuillet de couverture (11, 41) se projetant depuis la zone de collage de bord périphérique (15, 45) pour former ledit corps formant tirette (18,48).

4. La protection interlabiale (14, 44) selon la revendication 1 dans laquelle au niveau d'un bord périphérique de ladite protection interlabiale (14, 44), la surface côté vêtement dudit feuillet de couverture (11, 41), et la surface côté corps dudit feuillet formant couche dorsale (12, 42) sont collées pour former une zone de collage de bord périphérique (15, 45) de ladite protection interlabiale (14, 44), ledit feuillet formant couche dorsale (12, 42) se projetant depuis ladite zone de collage de bord périphérique (15, 45) pour former ledit corps formant tirette (18, 48).

5. La protection interlabiale (14, 44) selon n'importe laquelle des revendications 1 à 4 dans laquelle ladite portion d'ouverture (50b) ou ladite portion découpée formée dans ledit corps formant tirette (18, 48) est d'une conformation qui n'a pas de coin pointu.

6. La protection interlabiale (14, 44) selon n'importe laquelle des revendications 1 à 5 comprenant une zone comprimée destinée à contrôler le suintement de liquide formé à proximité d'une limite entre ledit corps formant tirette (18, 48) et ladite zone de collage de bord périphérique (15, 45).

7. La protection interlabiale (14, 44) selon n'importe laquelle des revendications 1 à 6 comprenant
un morceau formant mini-feuillet (52) collé dans au moins une zone de collage de chaque côté dans la direction longitudinale dudit feuillet formant couche dorsale (12, 42) sur le côté vêtement dudit feuillet formant couche dorsale (12, 42) et ayant au moins une zone non collée dans la direction latérale dudit feuillet formant couche dorsale (12, 42) ; et
une ouverture d'insertion de doigt (53) qui fournit une ouverture de la largeur d'un doigt dans la direction de surface dudit feuillet formant couche dorsale (12, 42) et est formée entre le morceau formant mini-feuillet (52) et le feuillet formant couche dorsale (12, 42) en utilisant ladite au moins une zone non collée.

8. La protection interlabiale (14, 44) selon la revendication 7 dans laquelle le corps formant tirette (18, 48) est une partie dudit morceau formant mini-feuillet (52).

9. Une protection interlabiale (14, 44) selon n'importe laquelle des revendications 1 à 8, la protection étant une protection interlabiale (14, 44) qui est utilisée conjointement avec une serviette hygiénique.

10. Une protection interlabiale (14, 44) selon n'importe laquelle des revendications 1 à 9, ladite protection interlabiale (14, 44) étant une protection destinée à l'incontinence.

11. Une protection interlabiale (14, 44) selon n'importe laquelle des revendications 1 à 9, ladite protection interlabiale (14, 44) étant une protection destinée à absorber des pertes vaginales.

12. Une protection interlabiale (14, 44) selon n'importe laquelle des revendications 1 à 11 dans laquelle ladite protection interlabiale (14, 44) est renfermée dans un étui d'emballage (56) destiné à emballer individuellement la protection interlabiale (14, 44) pour former un corps d'emballage (54).

13. Une protection interlabiale (14, 44) selon n'importe laquelle des revendications 7 à 12 dans laquelle ladite protection interlabiale (14, 44) est renfermée dans un étui d'emballage (56) destiné à emballer individuellement la protection interlabiale (14, 44) pour former un corps d'emballage (54), l'étui d'emballage (56) étant pourvu d'une portion d'ouverture, ladite protection interlabiale (14, 44) étant renfermée dans ledit étui d'emballage (56) de telle sorte que ladite ouverture d'insertion de doigt (53) s'ouvre vers ladite portion d'ouverture.

14. Une protection interlabiale (14, 44) telle que revendiquée dans la revendication 13 dans laquelle, lorsque la protection interlabiale (14, 44) est renfermée dans ledit étui d'emballage (66), le morceau formant mini-feuillet (52) est plié vers le côté vêtement le long d'une ligne substantiellement médiane dans la direction longitudinale de ladite protection interlabiale (14, 44).

15. Une protection interlabiale (14, 44) telle que revendiquée dans n'importe laquelle des revendications 1 à 14 dans laquelle la protection interlabiale (14, 44) est composée de matière biodégradable et / ou de matière dispersible dans l'eau et 1 ou de matière soluble dans l'eau.
